# FASCICULE DE BREVET EUROPEEN

(11) **EP 2 474 937 B1**
(45) Date de publication et mention de la délivrance du brevet: **10.12.2014**
(21) Numéro de dépôt: 12150739.6
(22) Date de dépôt: 11.01.2012
(51) Int. Cl.: G06K 9/00, G06F 3/044

(54) **Procédé d'authentification électronique d'une signature manuscrite, module et programme d'ordinateur correspondants**
Elektronisches Authentifizierungsverfahren einer handschriftlichen Unterschrift, Modul-und entsprechendes Computerprogramm
Electronic authentication method of a handwritten signature, module and corresponding computer program

(30) Priorité: 11.01.2011 FR 1150236
(43) Date de publication de la demande: 11.07.2012
(73) Titulaire: Compagnie Industrielle et Financière d'Ingénierie "Ingenico", 92200 Neuilly sur Seine (FR)
(72) Inventeur: Huteaux, Fabien, 92100 Boulogne-Billancourt (FR); Kroupski, Alexandre, 91600 Savigny-Sur-Orge (FR)
(74) Mandataire: Le Saux, Gaël

(56) Documents cités:
- US-A1- 2008 106 520
- US-B1- 6 486 874
- H B KEKRE ET AL: "Gabor Filter Based Feature Vector for Dynamic Signature Recognition", INTERNATIONAL JOURNAL OF COMPUTER APPLICATIONS, vol. 2, no. 3, 1 mai 2010 (2010-05-01), pages 74-80, XP055003987, DOI: 10.5120/639-895
- WON-CHUL BANG ET AL: "Self-contained spatial input device for wearable computers", WEARABLE COMPUTERS, 2003. PROCEEDINGS. SEVENTH IEEE INTERNATIONAL SYMP OSIUM ON 21-23 OCT. 2003, PISCATAWAY, NJ, USA,IEEE, LOS ALAMITOS, CA, USA, 21 octobre 2003 (2003-10-21), pages 26-34, XP010673827, DOI: DOI:10.1109/ISWC.2003.1241390 ISBN: 978-0-7695-2034-6
- ZHONGCHENG WU ET AL: "The Design of Digital Handwriting Forces Vector Ink and its Application in Online Signature Verification", INTELLIGENT ROBOTS AND SYSTEMS, 2006 IEEE/RSJ INTERNATIONAL CONFERENCE ON, IEEE, PI, 1 octobre 2006 (2006-10-01), pages 4239-4244, XP031006788, ISBN: 978-1-4244-0258-8
- HANGAI S ET AL: "On-line signature verification based on altitude and direction of pen movement", MULTIMEDIA AND EXPO, 2000. ICME 2000. 2000 IEEE INTERNATIONAL CONFEREN CE ON NEW YORK, NY, USA 30 JULY-2 AUG. 2000, PISCATAWAY, NJ, USA,IEEE, US, vol. 1, 30 juillet 2000 (2000-07-30), pages 489-492, XP010511502, DOI: DOI:10.1109/ICME.2000.869645 ISBN: 978-0-7803-6536-0
- PLAMONDON R ET AL: "AUTOMATIC SIGNATURE VERIFICATION AND WRITER IDENTIFICATION. ÖTHE STATE OF THE ART", PATTERN RECOGNITION, ELSEVIER, GB, vol. 22, no. 2, 1 mars 1989 (1989-03-01), pages 107-131, XP000461073, ISSN: 0031-3203, DOI: DOI:10.1016/0031-3203(89)90059-9

## Description

### 1. Domaine de l'invention

Le domaine de l'invention est celui de l'authentification de personnes, et notamment l'authentification électronique de signatures manuscrites de personnes.

Plus particulièrement, l'invention concerne l'authentification de signature manuscrite sur une surface d'écriture comprenant une zone tactile capacitive.

### 2. Art antérieur

A l'heure actuelle, il existe un certain nombre de techniques d'authentification de signatures manuscrites, basées essentiellement sur des mesures de corrélation entre des caractéristiques d'une signature à authentifier et des caractéristiques de référence de personnes autorisées, préalablement acquises.

Les techniques d'authentification actuelles peuvent être classées en deux grandes catégories : les techniques d'authentification « a posteriori » et les techniques d'authentification « temps réel ».

La première catégorie de techniques est basée sur une analyse de caractéristiques de la signature saisie (la taille et/ou la forme des lettres par exemple) ou de la surface d'écriture sur laquelle la signature a été saisie (reliefs créés par la saisie de la signature sur un document papier par exemple), après la saisie de la signature.

La deuxième catégorie de techniques est basée sur une analyse de caractéristiques de la signature obtenues au fur et à mesure de la saisie de la signature (la pression de l'instrument d'écriture sur la surface d'écriture et/ou l'accélération de l'instrument d'écriture pendant la signature par exemple), pouvant être combinées avec des caractéristiques de la première catégorie de techniques.

Cette deuxième catégorie de techniques nécessite la mise en oeuvre de moyens techniques pendant la saisie de la signature, en temps réel, comme par exemple la mise en oeuvre d'un accéléromètre sur l'instrument d'écriture, ou d'un capteur de pression sur la surface d'écriture.

Un des objectifs les plus importants à atteindre pour ces techniques d'authentification de signature manuscrite réside dans le niveau de sécurité fourni, ce dernier étant essentiellement lié au nombre de caractéristiques analysées.

En effet, plus le nombre de caractéristiques analysées est important, et plus l'authentification est fiable.

En revanche, plus le nombre de caractéristiques analysées est important, et plus les coûts de mise en oeuvre sont importants, les moyens d'obtention de ces caractéristiques devant être nombreux, ainsi que les moyens de traitement des caractéristiques obtenues, en vue de l'authentification ultérieure.

Ainsi, parmi les techniques actuelles, on observe que celles mises en oeuvre dans des produits « grand public » (tels que des tablettes graphiques) sont basées sur un nombre assez restreint de caractéristiques, pour des raisons de coûts, alors que celles mises en oeuvre dans des produits professionnels (tels que des tablettes de signature) sont basées sur un nombre assez important de caractéristiques, entraînant un coût de mise en oeuvre également important mais un niveau de sécurité supérieur.

Par exemple, il existe des techniques d'authentification « temps réel » basées sur l'analyse de caractéristiques telles qu'une représentation bidimensionnelle de la signature saisie, associée à un paramètre représentatif de la pression de l'instrument d'écriture sur la surface d'écriture et/ou à un paramètre représentatif de l'accélération de l'instrument d'écriture selon les trois axes de l'espace. Ces techniques sont relativement coûteuses car elles nécessitent la mise en oeuvre de capteurs à la fois sur la surface d'écriture et sur l'instrument d'écriture. Par ailleurs, elles nécessitent également des traitements complexes des différentes données captées, pour pouvoir ensuite effectuer les mesures de corrélation et ainsi authentifier la signature manuscrite saisie.

Un exemple de technique est "Gabor Filter Based Feature Vector for Dynamic Signature Recognition" par H.B. Kekre et V.A. Bharadi publié dans International Journal of Computer Applications (0975-8887) Volume 2 - No. 3, May 2010.

Un inconvénient des techniques actuelles d'authentification de signature manuscrite réside donc dans la résolution du compromis « coût-sécurité », c'est-à-dire dans le choix d'une complexité supérieure pour un niveau de sécurité supérieur et inversement.

Ainsi, il existe donc un besoin pour proposer une technique d'authentification électronique de signature manuscrite qui puisse présenter un niveau de sécurité optimal sans augmenter de façon importante la complexité ni le coût de mise en oeuvre.

### 3. Exposé de l'invention

L'invention propose une solution nouvelle qui ne présente pas l'ensemble de ces inconvénients de l'art antérieur, sous la forme d'un procédé d'authentification électronique d'une signature manuscrite d'un utilisateur, saisie sur une surface d'écriture via un instrument d'écriture.

Selon l'invention, un tel procédé comprend les étapes de la revendication 1.

Ainsi, l'invention permet de pallier les inconvénients de l'art antérieur en exploitant de manière simple et peu coûteuse une caractéristique « temps réel » d'une signature manuscrite représentative de l'altitude de l'instrument d'écriture au-dessus de la surface d'écriture.

Selon l'invention, le terme « altitude » correspond à la distance verticale variable séparant l'extrémité d'écriture de l'instrument d'écriture et la surface d'écriture.

Cette caractéristique se distingue de celles utilisées dans l'art antérieur par le fait qu'elle ne donne pas une information de type binaire indiquant si l'instrument d'écriture est en contact ou non avec la surface d'écriture, mais une information plus riche représentative de l'altitude, ou d'une variation de l'altitude, de l'instrument d'écriture par rapport à la surface d'écriture.

De plus, l'invention se distingue avantageusement des techniques de l'art antérieur par le mode d'acquisition de la donnée d'altitude. En effet, selon l'invention, cette donnée d'altitude est acquise par la surface d'écriture elle-même, sans aucune mise en oeuvre particulière au niveau de l'instrument d'écriture. De plus, cette mise en oeuvre nécessite peu ou pas de réglages préalables, ni aucune maintenance spécifique, contrairement aux techniques de l'art antérieur utilisant des capteurs optiques par exemple.

Ainsi, la mise en oeuvre de l'invention est simplifiée en termes techniques et permet donc de minimiser les coûts.

Par ailleurs, selon l'invention, l'authentification électronique de la signature manuscrite saisie par l'utilisateur tient notamment compte de la donnée d'altitude acquise par la surface d'écriture, renforçant ainsi le niveau de sécurité de l'authentification. En effet, cette caractéristique relative à l'altitude de l'instrument d'écriture fait partie des caractéristiques biométriques uniques pouvant servir à l'authentification d'un utilisateur via l'authentification de sa signature manuscrite.

Selon un aspect particulier de l'invention, l'étape d'acquisition met en oeuvre une mesure d'une capacité à l'aide d'une zone tactile capacitive de la surface d'écriture.

Ainsi, l'invention est mise en oeuvre dans un dispositif comprenant une surface d'écriture présentant au moins une zone tactile capacitive, permettant l'acquisition des données d'altitude lorsqu'un utilisateur saisit sa signature.

En effet, grâce aux technologies tactiles capacitives actuelles et futures, l'invention peut exploiter une caractéristique supplémentaire de la détection d'un instrument d'écriture sur une surface d'écriture de ce type, représentative de l'altitude de l'instrument d'écriture par rapport à la surface d'écriture, caractéristique distincte des coordonnées (x,y) pouvant être obtenues classiquement,

Selon un mode de réalisation particulier de l'invention, l'étape d'acquisition délivre en outre au moins deux autres données représentatives de la localisation de l'instrument d'écriture dans un plan formé par la surface d'écriture, notées données x et y.

Ainsi, selon ce mode de réalisation de l'invention, l'authentification d'une signature se base sur plusieurs autres caractéristiques, en plus de la donnée d'altitude, renforçant ainsi le niveau de sécurité.

Par exemple, les coordonnées (x, y, z) de l'instrument d'écriture sont acquises, permettant une localisation précise de l'instrument d'écriture sur et au-dessus de la surface d'écriture.

En particulier, les données x, y et z sont représentatives de la localisation de l'extrémité d'écriture de l'instrument d'écriture, c'est-à-dire l'extrémité se trouvant en contact ou proche au-dessus de la surface d'écriture (par exemple la pointe d'un stylet).

Selon un aspect particulier de l'invention, le procédé comprend une étape de traitement d'au moins une des données acquises lors de l'étape d'acquisition, comprenant au moins une des sous-étapes suivantes :
- filtrage ;
- linéarisation ;
- interpolation ;
- normalisation ;
- seuillage.

Ainsi, selon ce mode de réalisation, l'invention prévoit, le cas échéant, d'effectuer un traitement mathématique sur l'une des données acquises, notamment la donnée d'altitude. En effet, l'acquisition de cette donnée peut être imprécise, ou altérée, du fait qu'il peut ne pas y avoir de contact entre la surface d'écriture et l'instrument d'écriture. Ainsi, une donnée d'altitude acquise peut être par exemple « normalisée » après acquisition, de façon à être utilisable ensuite pour l'authentification de la signature manuscrite.

En particulier, la donnée d'altitude z appartient au groupe comprenant :
- une donnée représentative d'une mesure de l'altitude de l'instrument d'écriture par rapport à la surface d'écriture ;
- une donnée représentative d'une variation entre deux mesures de l'altitude de l'instrument d'écriture par rapport à la surface d'écriture.

Ainsi, l'invention permet non seulement d'acquérir une donnée représentative de l'altitude de l'instrument écriture, mais elle permet également d'acquérir une donnée représentative d'une évolution, ou d'une différence, d'altitude de l'instrument d'écriture, au fur et à mesure de la saisie de la signature. De cette manière, l'invention permet de connaître l'évolution de l'altitude de l'instrument d'écriture en fonction du temps, renforçant ainsi le niveau de sécurité.

Selon un mode de réalisation particulier de l'invention, l'étape d'acquisition comprend une sous-étape d'obtention d'une donnée représentative de l'altitude de l'instrument d'écriture mise en oeuvre périodiquement pendant la saisie de la signature manuscrite délivrant un ensemble de données représentatif du parcours de l'instrument d'écriture au-dessus de la surface d'écriture en fonction du temps.

Ainsi, une pluralité de données d'altitude peuvent être acquises au fur et à mesure de la saisie de la signature par l'utilisateur, permettant ainsi d'obtenir un ensemble de données représentatif du parcours de l'instrument d'écriture au-dessus de la surface d'écriture, en fonction du temps.

Par ailleurs, selon un autre aspect de l'invention, l'étape d'acquisition comprend une sous-étape de détermination d'une vitesse et/ou d'une accélération de l'instrument d'écriture.

En effet, une telle information de vitesse et/ou d'accélération est représentative de la spécificité d'une signature d'un utilisateur donné, et peut donc renforcer également le niveau de sécurité de l'authentification.

Selon un autre mode de réalisation de l'invention, le procédé comprend une phase préalable d'apprentissage comprenant les étapes suivantes :
- saisie, sur une surface d'écriture, via un instrument d'écriture, d'au moins une signature manuscrite dite de référence provenant d'un utilisateur autorisé ;
- acquisition, par la surface d'écriture, à au moins un instant de la saisie de la signature, d'au moins une donnée représentative de l'altitude z de l'instrument d'écriture par rapport à la surface d'écriture, dans une zone de proximité de la surface d'écriture, notée donnée d'altitude de référence ;
- stockage de la donnée d'altitude de référence.

Ainsi, le procédé prévoit une phase d'apprentissage, permettant de répertorier tous les utilisateurs autorisés, avec leur signature associée, servant de référence ensuite pour l'authentification.

Ces signatures de références peuvent par exemple être stockées dans un serveur d'authentification, distant d'un dispositif utilisé ensuite pour la saisie et l'authentification en temps réel d'une signature. Dans ce cas, au moment de l'authentification de la signature saisie sur un dispositif d'authentification, ce dernier interroge le serveur d'authentification pour avoir accès aux signatures de référence.

Les signatures de référence peuvent également être stockées dans le dispositif même servant ensuite à la saisie et l'authentification en temps réel d'une signature.

En particulier, l'étape d'authentification de la signature manuscrite comprend une sous-étape de mesure de corrélation entre au moins une donnée d'altitude z acquise lors de l'étape d'acquisition et au moins une donnée d'altitude de référence.

L'invention concerne également un module d'authentification électronique d'une signature manuscrite d'un utilisateur, saisie sur une surface d'écriture via un instrument d'écriture, caractérisé en ce qu'il met en oeuvre :
- des moyens d'acquisition, par la surface d'écriture, à au moins un instant de la saisie de la signature, d'au moins une donnée représentative de l'altitude z de l'instrument d'écriture par rapport à la surface d'écriture, dans une zone de proximité de la surface d'écriture, ladite altitude z correspondant à une distance verticale variable séparant l'extrémité d'écriture dudit instrument d'écriture et ladite surface d'écriture;
- des moyens d'authentification de la signature manuscrite saisie tenant compte d'au moins l'altitude z.

Un tel module d'authentification peut notamment faire partie d'un terminal de paiement électronique, d'un PDA ou encore d'un téléphone portable.

Enfin, l'invention concerne un programme d'ordinateur comportant des instructions pour l'exécution des étapes du procédé d'authentification tel que décrit précédemment, lorsque le programme est exécuté par un ordinateur.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- les figures 1A et 1B illustrent respectivement un exemple de système mettant en oeuvre le procédé d'authentification électronique d'une signature manuscrite et les principales étapes de ce procédé d'authentification, selon un mode de réalisation de l'invention ;
- les figures 2A et 2B illustrent respectivement un exemple de signature saisie dans un système selon la figure 1A et un exemple de données d'altitude z acquises selon un mode de réalisation du procédé d'authentification de l'invention.

### 5. Description d'un mode de réalisation de l'invention

### 5.1 Principe général

Le principe général de l'invention repose sur l'acquisition, en temps réel et par la surface d'écriture elle-même, d'au moins une donnée représentative de l'altitude d'un instrument d'écriture pendant la saisie d'une signature manuscrite d'un utilisateur, permettant de renforcer le niveau de sécurité d'une authentification de signature manuscrite basée sur la donnée d'altitude acquise, tout en minimisant les coûts d'implémentation.

En effet, la donnée d'altitude étant acquise par la surface d'écriture elle-même, l'invention ne nécessite pas de mise en oeuvre particulière au niveau de l'instrument d'écriture, peu ou pas de réglages ou de maintenance particulière, contrairement par exemple à l'utilisation de capteurs spécifiques optiques de certaines techniques de l'art antérieur.

De plus, la donnée d'altitude acquise peut être associée à un certain nombre d'autres caractéristiques de signature acquises en temps réel, afin de renforcer le niveau de sécurité de l'authentification de la signature. En effet, cette caractéristique relative à l'altitude de l'instrument d'écriture fait partie des caractéristiques biométriques uniques pouvant servir à l'authentification d'un utilisateur via l'authentification de sa signature manuscrite.

### 5.2 Description d'un mode de réalisation

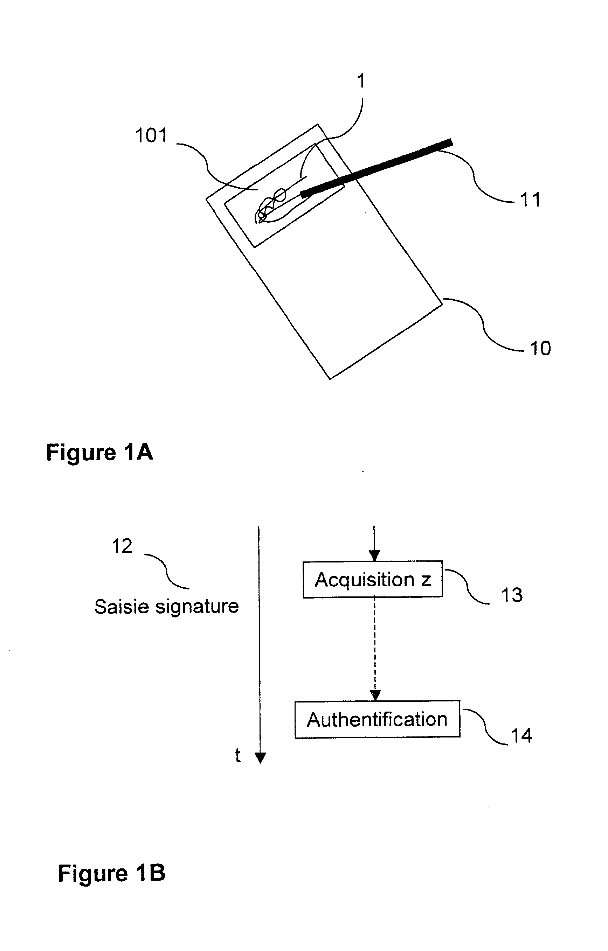

On présente maintenant, en relation avec les figures 1A et 1B, un exemple de système de mise en oeuvre de l'invention et les principales étapes du procédé d'authentification mis en oeuvre dans un tel système, selon un mode de réalisation de l'invention.

Comme illustré en figure 1A, on considère un module d'authentification 10 présentant une surface d'écriture 101 comprenant une zone tactile capacitive sur laquelle l'utilisateur saisit sa signature 1, en vue d'une authentification.

Par exemple, le module d'authentification est intégré dans un terminal de paiement, un téléphone mobile, un PDA, etc.

La signature 1 est saisie via un instrument d'écriture 11, adapté à une saisie sur la zone tactile capacitive, par exemple un stylet.

La figure 1B présente les principales étapes du procédé d'authentification d'une signature manuscrite, mis en oeuvre dans un système tel que décrit ci-dessus en relation avec la figure 1A

Une étape 13 d'acquisition d'une donnée z d'altitude de l'instrument d'écriture est mise en oeuvre, à au moins un instant de l'étape 12 de saisie de la signature manuscrite, et avantageusement à une pluralité d'instants au cours de la saisie de la signature.

Par exemple, on peut acquérir trois cent échantillons par seconde au cours de la saisie de la signature, pour obtenir des bonnes performances en terme de fiabilité d'authentification.

Par ailleurs, selon le type de zone tactile capacitive, on peut acquérir des données d'altitude jusqu'à deux centimètres au-dessus de la surface d'écriture.

Selon une variante avantageuse de ce mode de réalisation, l'étape d'acquisition permet d'acquérir une pluralité d'autres données relatives à la position de l'instrument d'écriture par rapport à la zone tactile capacitive de la surface d'écriture, comme par les coordonnées (x,y) de l'instrument d'écriture sur la surface d'écriture, à plusieurs moments de la signature. Des données de pression sur la surface d'écriture peuvent également être acquises. Cette variante permet de renforcer l'authentification en fournissant un plus grand nombre de caractéristiques « temps réel » de la signature.

Il est à noter que les coordonnées x, y et z sont acquises par la zone tactile capacitive de la surface d'écriture elle-même, sans nécessiter de mise en oeuvre particulière au niveau de l'instrument d'écriture. Par ailleurs, cette mise en oeuvre par la surface d'écriture elle-même ne nécessite pas non plus de réglages, ou peu de réglages, ni de maintenance particulière, contrairement à l'utilisation de capteurs optiques sur l'instrument d'écriture par exemple.

Après l'étape d'acquisition 13, ou au fur et à mesure de l'acquisition des données d'altitude z, une étape d'authentification 14 est mise en oeuvre, délivrant un résultat, positif ou négatif, d'authentification de l'utilisateur ayant saisi la signature.

Selon une première variante, l'étape d'authentification 14 peut être mise en oeuvre après la saisie de la signature, et donc après l'acquisition de toutes les données d'altitude. Cette variante est par exemple mise en oeuvre lorsque les données de référence servant à l'authentification sont disponibles sur un serveur distant, avec lequel il faut initier une communication pour pouvoir utiliser ces données de référence.

Selon une autre variante, l'authentification peut être mise en oeuvre dès qu'un nombre prédéterminé de données est acquis, de façon à permettre une authentification rapide. Par exemple, cette variante est mise en oeuvre lorsque les données de référence sont disponibles sur le module de saisie de la signature lui-même, ne nécessitant ainsi pas de communication avec un serveur distant.

Selon ce mode de réalisation, l'étape d'authentification 14 met en oeuvre une corrélation entre les données acquises (données d'altitude z, et éventuelles autres données décrites ci-dessus) pendant la saisie de la signature et des données de référence disponibles, répertoriées pour un certain nombre d'utilisateurs autorisés, lors d'une phase d'apprentissage.

Par exemple, on utilise des techniques de corrélation connues, non décrites ici, élargies à la corrélation de données d'altitude acquises selon l'invention. Ainsi, il existe des techniques de corrélation basées sur un ensemble de données (x, y, p, t) représentant les coordonnées (x,y) de l'instrument d'écriture et la pression sur la surface d'écriture, en fonction du temps. Selon une variante de l'invention, on peut donc élargir ces techniques de corrélation à un ensemble de données (x, y, z, t) ou un ensemble de données (x, y, z, p, t), pour encore plus de sécurité.

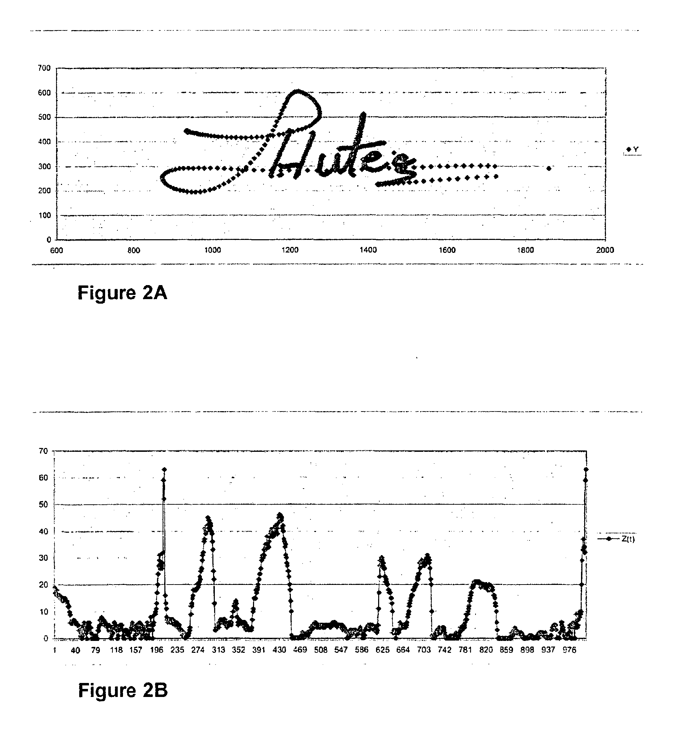

On décrit maintenant, en relation avec les figures 2A et 2B, respectivement un exemple d'une telle signature saisie ainsi qu'une représentation, en fonction du temps, d'une pluralité de données d'altitude z acquises au cours de la saisie de la signature.

Selon des variantes de ce mode de réalisation, particulièrement liées au type de la zone tactile capacitive utilisée comme surface d'écriture, les données d'altitude z acquises lors de la saisie de la signature peuvent être traitées avant d'être utilisées pour l'authentification de l'utilisateur.

Par exemple, selon une première variante, les données d'altitude z acquises sont normalisées selon des critères prédéterminés, relatifs au type de zone tactile capacitive. De cette manière, les données d'altitude normalisées peuvent ensuite être utilisées lors de l'authentification de la signature, même si les données d'altitude de référence n'ont pas été acquises avec un dispositif présentant le même type de zone tactile capacitive. Cette variante permet d'assurer une compatibilité d'authentification quel que soit le dispositif de saisie de la signature.

Selon une autre variante, on définit un espace, au-dessus de la zone tactile capacitive, linéaire et contigu de mesure de donnée d'altitude, tenant compte des défauts éventuels d'acquisition de données d'altitude. Cette variante permet ainsi d'obtenir des données d'altitude fiables et utilisables, tenant compte d'éventuelles imprécisions d'acquisition.

La figure 2B illustre un exemple de pluralité de données d'altitude acquises lors le saisie de la signature illustrée en figure 2A. Ces données d'altitude sont exploitables pour une authentification d'une signature manuscrite, c'est-à-dire aptes à être corrélées à une pluralité de données d'altitude de référence préalablement acquises lors d'une phase d'apprentissage.

Selon encore une autre variante de ce mode de réalisation, compatibles avec les variantes précédemment décrites, les données d'altitude acquises sont traitées de façon à obtenir une représentation de l'évolution de l'altitude de l'instrument d'écriture au fur et à mesure de la signature. Ainsi, on obtient des données relatives d'altitude de l'instrument d'écriture, à corréler ensuite à des données relatives d'altitude de référence. Cette variante permet de mieux tenir compte de l'évolution globale de l'altitude de l'instrument d'écriture pendant la saisie de la signature.

## Revendications

1. **Procédé** d'authentification électronique d'une signature manuscrite (1) d'un utilisateur, saisie sur une surface d'écriture (101) via un instrument d'écriture (11), **caractérisé en ce qu'**il comprend :
- une étape d'acquisition (13), par ladite surface d'écriture, à au moins un instant de la saisie (12) de ladite signature, d'au moins une donnée représentative de l'altitude z dudit instrument d'écriture (11) par rapport à ladite surface d'écriture (101), dans une zone de proximité de ladite surface d'écriture (101), ladite altitude z correspondant à une distance verticale variable séparant l'extrémité d'écriture dudit instrument d'écriture et ladite surface d'écriture, ladite étape d'acquisition (13) mettant en oeuvre une mesure d'une capacité à l'aide d'une zone tactile capacitive de ladite surface d'écriture (101) ;
- une étape d'authentification (14) de ladite signature manuscrite saisie (1) tenant compte d'au moins ladite altitude z.

2. **Procédé** d'authentification selon la revendication 1, **caractérisé en ce que** ladite étape d'acquisition délivre en outre au moins deux autres données représentatives de la localisation dudit instrument d'écriture dans un plan formé par ladite surface d'écriture, notées données x et y.

3. **Procédé** d'authentification selon la revendication 2, **caractérisé en ce que** lesdites données x, y et z sont représentatives de la localisation de l'extrémité d'écriture dudit instrument d'écriture.

4. **Procédé** d'authentification selon la revendication 1, **caractérisé en ce qu'**il comprend une étape de traitement d'au moins une desdites données acquises lors de ladite étape d'acquisition, comprenant au moins une des sous-étapes suivantes :
- filtrage ;
- linéarisation ;
- interpolation ;
- normalisation ;
- seuillage.

5. **Procédé** d'authentification selon la revendication 1, **caractérisé en ce que** ladite donnée d'altitude z appartient au groupe comprenant :
- une donnée représentative d'une mesure, à un instant de la saisie de ladite signature, de l'altitude dudit instrument d'écriture par rapport à ladite surface d'écriture (101) ;
- une donnée représentative d'une variation entre deux mesures, à deux instants de ladite signature, de l'altitude dudit instrument d'écriture par rapport à ladite surface d'écriture (101).

6. **Procédé** d'authentification selon la revendication 1, **caractérisé en ce que** ladite étape d'acquisition comprend une sous-étape d'obtention d'une donnée représentative de l'altitude dudit instrument d'écriture mise en oeuvre périodiquement pendant la saisie de ladite signature manuscrite délivrant un ensemble de données représentatif du parcours dudit instrument d'écriture au-dessus de ladite surface d'écriture en fonction du temps.

7. **Procédé** d'authentification selon la revendication 5 et la revendication 6, **caractérisé en ce que** ladite étape d'acquisition comprend une sous-étape de détermination d'une vitesse et/ou d'une accélération dudit instrument d'écriture.

8. **Procédé** d'authentification selon la revendication 1, **caractérisé en ce qu'**il comprend une phase préalable d'apprentissage comprenant les étapes suivantes :
- saisie, sur une surface d'écriture, via un instrument d'écriture, d'au moins une signature manuscrite dite de référence provenant d'un utilisateur autorisé ;
- acquisition, par ladite surface d'écriture, à au moins un instant de la saisie de ladite signature, d'au moins une donnée représentative de l'altitude z dudit instrument d'écriture par rapport à ladite surface d'écriture, dans une zone de proximité de ladite surface d'écriture, notée donnée d'altitude de référence ;
- stockage de ladite au moins une donnée d'altitude de référence.

9. **Procédé** d'authentification électronique selon la revendication 8, **caractérisé en ce que** ladite étape d'authentification de ladite signature manuscrite comprend une sous-étape de mesure de corrélation entre ladite au moins une donnée d'altitude z acquise lors de ladite étape d'acquisition et au moins une donnée d'altitude de référence.

10. **Module** d'authentification électronique d'une signature manuscrite (1) d'un utilisateur, saisie sur une surface d'écriture (101) via un instrument d'écriture (11), **caractérisé en ce qu'**il met en oeuvre :
- des moyens d'acquisition (13), par ladite surface d'écriture, à au moins un instant de la saisie (12) de ladite signature, d'au moins une donnée représentative de l'altitude z dudit instrument d'écriture (11) par rapport à ladite surface d'écriture (101), dans une zone de proximité de ladite surface d'écriture (101), ladite altitude z correspondant à une distance verticale variable séparant l'extrémité d'écriture dudit instrument d'écriture et ladite surface d'écriture, lesdits moyens d'acquisition mettant en oeuvre une mesure d'une capacité à l'aide d'une zone tactile capacitive de ladite surface d'écriture (101) ;
- des moyens d'authentification (14) de ladite signature manuscrite saisie (1) tenant compte d'au moins ladite altitude z.

11. Programme d'ordinateur comportant des instructions pour l'exécution des étapes du procédé d'authentification selon l'une quelconque des revendications 1 à 9, lorsque ledit programme est exécuté par un ordinateur.

## Patentansprüche

1. Verfahren zur elektronischen Authentifizierung einer handschriftlichen Unterschrift (1) eines Benutzers, die auf einer Schreibfläche (101) mittels eines Schreibinstruments (11) erfasst wurde, **dadurch gekennzeichnet, dass** es folgendes aufweist:
- einen Schritt zum Beschaffen (13) über die besagte Schreibfläche, zu mindestens einem Zeitpunkt des Erfassens (12) der besagten Unterschrift, von mindestens einem für die Höhe z des besagten Schreibinstrumentes (11) im Verhältnis zur besagten Schreibfläche (101) repräsentativen Datenwertes innerhalb eines Nahbereiches der besagten Schreibfläche, wobei die besagte Höhe z einen variablen vertikalen Abstand entspricht, welcher das Schreibende des besagten Schreibinstrumentes von der besagten Schreibfläche trennt, wobei der besagte Beschaffungsschritt (13) eine Kapazitätsmessung mit Hilfe eines kapazitätssensitiven Bereichs der besagten Schreibfläche (101) einsetzt;
- einen Schritt zur Authentifizierung (14) der besagten erfassten handschriftlichen Unterschrift (1), der mindestens die besagte Höhe z berücksichtigt.

2. Authentifizierungsverfahren nach Anspruch 1, **dadurch gekennzeichnet, dass** der besagte Beschaffungsschritt darüber hinaus mindestens zwei andere Datenwerte liefert, die für die Lokalisierung des besagten Schreibinstrumentes in einer Ebene repräsentativ sind, welche von der besagten Schreibfläche gebildet wird und die mit x und y bezeichnet werden.

3. Authentifizierungsverfahren nach Anspruch 2, **dadurch gekennzeichnet, dass** die besagten Datenwerte x, y und z für die Lokalisierung des Schreibendes des besagten Schreibinstrumentes repräsentativ sind.

4. Authentifizierungsverfahren nach Anspruch 1, **dadurch gekennzeichnet, dass** es einen Schritt zum Bearbeiten von mindestens einem der Datenwerte aufweist, die während des besagten Beschaffungsschrittes erhalten wurden und mindestens einen der folgenden Unterschritte aufweist:
- Filterung;
- Linearisierung;
- Interpolation;
- Normierung;
- Ausführung eines Schwellenwertverfahrens.

5. Authentifizierungsverfahren nach Anspruch 1, **dadurch gekennzeichnet, dass** der besagte Höhendatenwert z der Gruppe angehört, die folgendes umfasst:
- einen Datenwert, der für eine Messung der Höhe des besagten Schreibinstrumentes im Verhältnis zur besagten Schreibfläche (101) zu einem Zeitpunkt des Erfassens der besagten Unterschrift repräsentativ ist;
- einen Datenwert, der für eine Variation zwischen zwei Messungen zu zwei Zeitpunkten der besagten Unterschrift für die Höhe des besagten Schreibinstrumentes im Verhältnis zu der besagten Schreibfläche (101) repräsentativ ist.

6. Authentifizierungsverfahren nach Anspruch 1, **dadurch gekennzeichnet, dass** der besagte Beschaffungsschritt einen Unterschritt zum Erlangen eines Datenwertes aufweist, der für die Höhe des besagten Schreibinstrumentes repräsentativ ist, wobei dieser Schritt periodisch während der Erfassung der besagten Unterschrift eingesetzt wird und eine Menge von Daten liefert, die für den von dem besagten Schreibinstrument zurückgelegten Weg oberhalb der besagten Schreibfläche als Funktion der Zeit repräsentativ sind.

7. Authentifizierungsverfahren nach Anspruch 5 und nach Anspruch 6, **dadurch gekennzeichnet, dass** der besagte Beschaffungsschritt einen Unterschritt zum Bestimmen einer Geschwindigkeit und/oder einer Beschleunigung des besagten Schreibinstrumentes aufweist.

8. Authentifizierungsverfahren nach Anspruch 1, **dadurch gekennzeichnet, dass** es eine vorhergehende Lernphase aufweist, welche die folgenden Schritte umfasst:
- Erfassen mindestens einer, Referenzunterschrift genannten, handschriftlichen Unterschrift eines zugelassenen Benutzers auf einer Schreibfläche, mit Hilfe eines Schreibinstrumentes;
- Beschaffung durch die besagte Schreibfläche, zu mindestens einem Zeitpunkt des Erfassens der besagten Unterschrift, von mindestens einem Datenwert, der für die Höhe z des besagten Schreibinstrumentes im Verhältnis zur besagten Schreibfläche repräsentativ ist, innerhalb eines Nahbereiches der besagten Schreibfläche, der als Referenzhöhenwert bezeichnet wird;
- Speichern des besagten mindestens einen Referenzhöhenwertes.

9. Elektronisches Authentifizierungsverfahren nach Anspruch 8, **dadurch gekennzeichnet, dass** der besagte Authentifizierungsschritt der besagten handschriftlichen Unterschrift einen Unterschritt zum Messen der Korrelation zwischen dem während dem besagten Beschaffungsschritt erhaltenen besagten mindestens einen Höhenwert z und mindestens einen Höhenreferenzwert, umfasst.

10. Elektronisches Authentifizierungsmodul einer handschriftlichen Unterschrift (1) eines Benutzers, die auf einer Schreibfläche (101) mittels eines Schreibinstruments (11) erfasst wird, **dadurch gekennzeichnet, dass** es folgendes zum Einsatz bringt:
- Mittel zum Beschaffen (13) durch die besagte Schreibfläche, zu mindestens einem Zeitpunkt des Erfassens (12) der besagten Unterschrift, von mindestens einem für die Höhe z des besagten Schreibinstrumentes (11) im Verhältnis zur besagten Schreibfläche (101) repräsentativen Datenwertes, innerhalb eines Nahbereiches der besagten Schreibfläche (101), wobei die besagte Höhe z einer variablen vertikalen Abstand entspricht, welcher das Schreibende des besagten Schreibinstrumentes von der besagten Schreibfläche trennt, wobei die besagten Beschaffungsmittel eine Kapazitätsmessung mit Hilfe eines kapazitätssensitiven Bereiches der besagten Schreibfläche (101) einsetzt;
- Mittel zur Authentifizierung (14) der besagten erfassten handschriftlichen Unterschrift (1), welche mindestens die besagte Höhe z berücksichtigen.

11. EDV-Programm, das Anweisungen für die Durchführung der Schritte des Authentifizierungsverfahrens nach einem der Ansprüche 1 bis 9 umfasst, wenn das besagte Programm von einem Rechner ausgeführt wird.

## Claims

1. **Method** for the electronic authenticating of a handwritten signature (1) of a user entered on a writing surface (101) via a writing instrument (11), **characterized in that** it comprises:
- a step of acquisition (13), by said writing surface, at one instant at least of the entering or entry (12) of said signature, of at least one piece of data representing the altitude z of said writing instrument (11) relatively to said writing surface (101), in an area of proximity to said writing surface (101), said step of acquisition (13) implementing a measurement of a capacitance by means of a capacitive touch area of said writing surface (101);
- a step of authenticating (14) said entered handwritten signature that takes account of at least said altitude z.

2. **Method** for authenticating according to claim 1, **characterized in that** said acquisition step furthermore delivers at least two other pieces of data representing the localization of said writing instrument in a plane formed by said writing surface, denoted as pieces of data x and y.

3. **Method** for authenticating according to claim 2, **characterized in that** said pieces of data x, y and z represent the localization of the writing end of said writing instrument.

4. **Method** for authenticating according to claim 1, **characterized in that** it comprises a step for processing at least one of said pieces of data acquired during said acquisition step, comprising at least one of the following sub-steps:
- filtering;
- linearization;
- interpolation;
- standardization;
- thresholding.

5. **Method** for authenticating according to claim 1, **characterized in that** said piece of data on altitude z belongs to the group comprising:
- a piece of data representing a measurement, at an instant of the entering or entry (12) of said signature, of the altitude of said writing instrument relatively to said writing surface (101);
- a piece of data representing a variation between two measurements, at two instants of said signature, of the altitude of said writing instrument.

6. **Method** for authenticating according to claim 1, **characterized in that** said acquisition step is implemented periodically during the entering or entry of said handwritten signature.

7. **Method** for authenticating according to claim 5 and claim 6, **characterized in that** said acquisition step comprises a sub-step for determining a speed and/or an acceleration of said writing instrument.

8. **Method** for authenticating according to claim 1, **characterized in that** it comprises a preliminary learning phase comprising the following steps:
- the entering or entry, on a writing surface through a writing instrument, of at least one handwritten signature called a reference signature coming from an authorized user;
- the acquisition, by said writing surface, at one instant at least of the entering or entry of said signature, of at least one piece of data representing the altitude z of said writing instrument relatively to said writing surface in an area of proximity to said writing surface, denoted as a piece of reference altitude data;
- the storage of said at least one piece of reference altitude data.

9. **Method** for electronic authenticating according to claim 8, **characterized in that** said step for authenticating said handwritten signature comprises a sub-step for measuring the correlation between said at least one piece of data on altitude z acquired during said acquisition step and at least one piece of reference altitude data.

10. **Module** for the electronic authenticating of a handwritten signature (1) of a user, entered on a writing surface (101) via a writing instrument (11), **characterized in that** it implements:
- means of acquisition (13), by said writing surface, at one instant at least of the entering or entry (12) of said signature, of at least one piece of data representing the altitude z of said writing instrument (11) relatively to said writing surface in an area of proximity to said writing surface (101), said means of acquisition (13) implementing a measurement of a capacitance by means of a capacitive touch area of said writing surface (101);
- means for authenticating (14) said handwritten signature taking account of at least said altitude z.

11. Computer program comprising instructions to execute the steps of the method for authenticating according to any one of the claims 1 to 9, when said program is executed by a computer.
